(19) Europäisches Patentamt
European Patent Office
Office européen des brevets

(11) Publication number: 0 085 511
B1

(12) **EUROPEAN PATENT SPECIFICATION**

(45) Date of publication of the patent specification: **12.03.86**

(51) Int. Cl.⁴: **C 07 C 91/44**, C 07 C 83/02

(21) Application number: **83300298.3**

(22) Date of filing: **20.01.83**

(54) **Process for preparing p-aminophenol and alkyl substituted p-aminophenol.**

(30) Priority: **29.01.82 US 343996**

(43) Date of publication of application: **10.08.83 Bulletin 83/32**

(45) Publication of the grant of the patent: **12.03.86 Bulletin 86/11**

(84) Designated Contracting States: **AT BE CH DE FR GB IT LI LU NL SE**

(56) References cited:
**DE - A - 2 118 334**
**US - A - 3 383 416**
**US - A - 3 654 365**
**US - A - 3 953 509**

**CHEMICAL ABSTRACTS, vol. 85, no. 19, November 8, 1976, page 499, no. 142816z, Columbus, Ohio, US**
**CHEMICAL ABSTRACTS, vol. 92, no. 11, March 17, 1980, page 559, no. 94060u, Columbus, Ohio, US**

(73) Proprietor: **MALLINCKRODT, INC., 675 McDonnell Boulevard P.O. Box 5840, St. Louis Missouri 63134 (US)**

(72) Inventor: **Caskey, Douglas C., 9 Belleau Lake Court O'Fallon, Missouri 63366 (US)**
Inventor: **Chapman, Douglas W., 48 Greendale Drive St. Louis, Missouri 63121 (US)**

(74) Representative: **Eyles, Winifred Joyce et al, BATCHELLOR, KIRK & EYLES 2 Pear Tree Court Farringdon Road, London EC1R 0DS (GB)**

**EP 0 085 511 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the field of the preparation of substituted or unsubstituted p-aminophenol and, more particularly, to an improved process for the manufacture of substituted or unsubstituted p-aminophenol by catalytic hydrogenation.

Various processes are known to the art for the manufacture of p-aminophenol. In the process described in Benner U.S. Patent 3,383,416, nitrobenzene is catalytically hydrogenated in the presence of a dilute sulfuric acid solution. Under the conditions utilized in the Benner patent, nitrobenzene is subject to complete reduction to aniline by reaction with three moles of hydrogen.

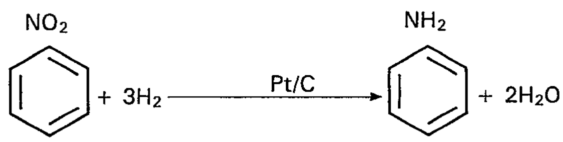


However, the intermediate phenylhydroxylamine, produced by reaction of nitrobenzene with two moles of hydrogen, is rapidly removed from the reaction zone by absorption into the sulfuric acid phase where it rearranges to p-aminophenol:

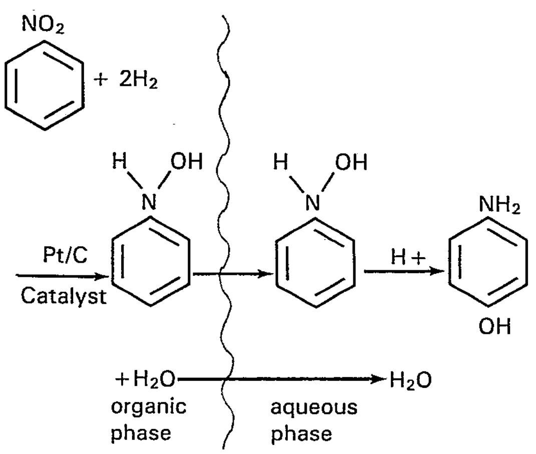


Fortuitously, the noble metal catalyst tends to congregate in the nitrobenzene phase so that phenylhydroxylamine absorbed into the acid phase is essentially removed from the hydrogenation reaction zone, and conversion of phenylhydroxylamine to aniline is thereby inhibited. This feature also permits recovery of the catalyst in the nitrobenzene phase when the phases are permitted to separate so that, by terminating the reaction prior to the conversion of all the nitrobenzene, the catalyst is easily recovered by decantation and recycled in its nitrobenzene vehicle for use in another batch, or in an earlier stage of a cascade continuous hydrogenator.

Despite its numerous salutary features, the Benner process does not entirely avoid conversion of phenylhydroxylamine to aniline. Thus, typically the yield of p-aminophenol is about 75% while 15% of the nitrobenzene is reduced to aniline. The amount of by-product aniline obtained can be minimized by lowering the reaction rate, but this, of course, involves a sacrifice in productivity.

Rylander et al. U.S. Patent 3,715,397 and corresponding German Specification No. 2118334 disclose a process for preparation of p-aminophenol by catalytic hydrogenation of nitrobenzene using a platinum oxide catalyst in the presence of sulfuric acid and dimethyl sulfoxide. Phenylhydroxylamine produced in the hydrogenation is absorbed into the acid phase and converted in situ to p-aminophenol. The acid strength is that obtained by mixing 25 ml. concentrated sulfuric acid with 75 ml. water. Reaction is carried out in a batch system until absorption of two moles of hydrogen per mole of nitrobenzene. According to the Rylander et al. disclosure, the reaction is preferably carried out at room temperature using comparatively large amounts of dimethyl sulfoxide modifier.

In the co-pending and co-assigned application of Thomas H. McFadden, a process is described in which dimethyl sulfoxide or another organic sulfoxide is incorporated into a continuous process for the manufacture of p-aminophenol by catalytic hydrogenation of nitrobenzene. In the McFadden process, 50 to 90 percent of the nitrobenzene charged to the first of three cascade hydrogenators is reduced leaving an unreacted nitrobenzene fraction which is allowed to separate from the reaction mixture and in which the catalyst congregates. After separation, the nitrobenzene phase containing the catalyst is recycled to the first hydrogenator.

The present invention seeks to provide an improved process for the manufacture of p-aminophenol or alkyl substituted p-aminophenols by catalytic hydrogenation of nitrobenzene or an alkyl substituted nitrobenzene; to provide such a process which minimizes the formation of by-product aniline; to provide such a process which facilitates production of substituted or unsubstituted p-aminophenol in high yield and at high productivity; to provide such a process which improves the selectivity of the catalyst for p-aminophenol and which inhibits formation of by-product aniline by addition of relatively low proportions of catalyst modifiers; and to provide such a process which provides improved catalytic activity without adverse effect on the partial pressure of hydrogen available for driving the reduction reaction.

Briefly, therefore, the present invention is directed to a process for preparation of a substituted or unsubstituted p-aminophenol having the formula:

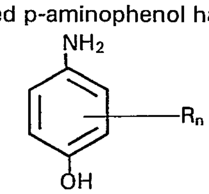

where R is lower alkyl and n is 0, 1 or 2. The process comprises preparing a charge mixture comprising a substituted or unsubstituted nitrobenzene substrate having the formula:

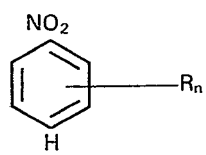

where R and n are as defined above, a highly dissociated acid, a catalyst containing platinum, and a sulfur compound. The sulfur compound is one in which divalent sulfur is bonded to two other moieties, and it is present in a proportion of between 0.1 and 100 moles per gram-atom platinum. The mixture comprises an organic phase containing the substrate and an aqueous phase containing the acid. Hydrogen is introduced into the mixture at a pressure of from 1 to 4.447 bar (0 to 50 psig) while the mixture is agitated at a temperature of between 70° and 100°C, thereby causing the substrate to be reduced to an intermediate comprising a substituted or unsubstituted phenylhydroxylamine having the structural formula:

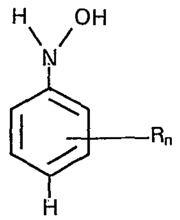

where R and n are as defined above. The intermediate is absorbed into the aqueous phase and therein rearranged to p-aminophenol or an alkyl substituted p-aminophenol corresponding to the substrate. The product is recovered from the aqueous phase.

The single figure of the drawings is a schematic flow sheet illustrating an embodiment in which the process of the invention is carried out continuously in a cascade hydrogenator system.

In accordance with the present invention, it has been discovered that improvements in productivity and yield may be achieved by using a divalent sulfur compound to modify the catalyst system used in the preparation of p-aminophenol by hydrogenation of nitrobenzene in the presence of a highly dissociated acid. We have found that use of a sulfur compound in such a system essentially eliminates conversion of nitrobenzene to by-product aniline, and thus enhances both the yields and productivity achievable in the hydrogenation process. Yield improvement is realised not only with respect to the substrate, but also with respect to consumption of hydrogen.

Use of a sulfur compound catalyst modifier allows application of higher hydrogen pressures than those permitted by the Benner process and thus affords opportunity for higher productivities. In the Benner process, pressures in excess of 5 psig favour aniline formation and are, thus preferably avoided. In the process of the invention, the use of a sulfur compound inhibits aniline formation and allows operation at pressures up to 4.447 bar (50 psig) or higher.

It has been further found that divalent sulfur compounds in which sulfur is bonded to two other moieties provide more effective catalyst selectivity enhancement than does the dimethyl sulfoxide taught by Rylander et al. While the charge mixture may contain between about 0.1 and 100 moles sulfur compound per gram atom of platinum, normally a proportion in the lower end of that range is fully adequate for achieving the desired catalyst selectivity.

Among the various sulfur compounds which can be used in the process of the invention are dialkyl sulfides, diaryl sulfides, dialkyl disulfides, aryl alkyl sulfides, thiophene, substituted thiophenes, thiophane, substituted thiophanes, aliphatic thiols, aromatic thiols, methionine and hydrogen sulfide. It is strongly preferred that the sulfur compound be substantially nonvolatile, i.e., that, under the conditions of the hydrogenation reaction, volatility not be such as to lower the hydrogen partial pressure so much as to adversely effect productivity. Although, by this criterion the suitability of the particular sulfur compounds may vary with the total available hydrogen pressure, it is particularly preferred that the sulfur compound used be one which causes no penalty in productivity in a continuous system at an available hydrogen pressure of 4.447 bar (50 psig). Generally, results are satisfactory if the sulfur compound has an atmospheric boiling point not lower than the temperature at which the hydrogenation is conducted.

While the partial pressure of sulfide compound in the vapor space of the hydrogenator varies with the proportion of sulfide compound in the reacting mixture, it has been found that the vapor pressure of dimethylsulfide is high enough to inhibit reaction at 80°C where the total available hydrogen pressure is in the 50 psig range. Thus, dimethylsulfide, hydrogen sulfide and other relatively volatile sulfur compounds are preferably avoided, even though they are effective for the purpose for enhancing the selectivity of the catalyst. Diethyl sulfide has a volatility low enough to be satisfactory in most circumstances, while diisopropyl sulfide and di(n-butyl)sulfide have even lower vapor pressures and are highly suitable.

In the preferred embodiment of the invention, only 50-90% of the nitrobenzene is reduced in the catalytic hydrogenation. At temperatures adequate for the rearrangement reaction, complete conversion promotes the formation of by-products that poison the catalyst. This effect rends to be aggravated by the presence of the sulfur compound. However, by operating in the 50-90% conversion range the aggravating effect of sulfur compound on catalyst poisoning is avoided or minimized.

Illustrated in the drawing is a schematic flow sheet of an embodiment in which the process of the invention is carried out continuously in a three hydrogenator cascade system. Fresh nitrobenzene, dilute highly dissociated acid (preferably sulfuric), water, sulfur compound and catalyst are charged to a first hydrogenation reactor 1 along with a recycle stream of unreacted nitrobenzene and a catalyst. A cationic surfactant is also preferably included in the charge mixture. Hydrogen is introduced into reactor 1 via a hydrogen supply line 2 and the hydrogenation reaction is carried out under a hydrogen pressure of 0 to 50 psig by agitation of the mixture contained in the vessel at the temperature in the range of 70-100°C, preferably 77-82°C. Nitrobenzene is readily reduced to phenylhydroxylamine but the presence of the sulfur compound inhibits conversion of phenylhydroxylamine to aniline and, as the phenylhydroxylamine is formed, it is absorbed into the aqueous phase. There, it rearranges to p-aminophenol in the presence of the acid. Exothermic reaction heat from the reduction and rearrangement reactions is removed and the reaction temperature controlled by cooling water passed through a jacket, internal coil or external heat exchanger associated with the reactor. The use of a temperature above room temperature (as illustrated by Rylander) increases the rate of the rearrangement reaction and minimizes the production of unwanted o-aminophenol.

Reacting mixture continuously overflows reactor 1 through a discharge line 3 to a second reactor 5 which is also agitated under hydrogen pressure at a temperature in the aforesaid range. Reduction of nitrobenzene and formation of p-aminophenol continues in reactor 5 and exothermic reaction heat is removed in the same manner as for reactor 1. Because a greater share of the reaction occurs in reactor 1, the cooling load on reactor 5 is significantly lower than for reactor 1. Reacting mixture continuously overflows from reactor 5 through an overflow line 7 into a third reactor 9 where the reaction continues at a rate somewhat below that occurring in reactor 5. The temperature of the reaction mixture in reactor 9 is controlled at approximately the same level as in reactors 1 and 5. Temperature control is provided in essentially the same manner as for the other reactors but, depending on the degree of conversion in the first two reactors, relatively little cooling may be needed in the third reactor.

By coordinated control of fresh nitrobenzene feed rate, catalyst loading, hydrogen pressure and temperature, conversion of nitrobenzene in the reactor system is preferably limited to between about 50% and about 90%. Optimal conversion is typically about 80%.

Reaction mixture overflows continuously from reactor 9 through a discharge pipe 11 into a decant tank 13. In the decant tank, the phases separate, with unreacted nitrobenzene organic phase containing the catalyst settling to the bottom and the aqueous phase containing p-aminophenol and acid rising to the top. Nitrobenzene and catalyst are continuously drawn off the bottom of decant tank 13 and returned to reactor 1. P-aminophenol liquor (aqueous phase) overflows continuously from the top of decant tank 13 and is subsequently subjected to conventional carbon treatment and precipitation steps for purification and recovery of the final product.

Although sulfuric acid is the preferred agent for the rearrangement reaction, other dilute highly dissociated acids may be used. Benzenesulfonic acid and toluenesulfonic acid are particularly suitable but other acids such as phosphoric acid and trihaloacetic are effective. Hydrogen halide acids are preferably not used.

When sulfuric acid is used, the dilute acid typically has substantially the same composition as disclosed in the Benner patent, i.e., 5-20% by weight, preferably 10-15% by weight. However, the relative proportions of acid to nitrobenzene may typically differ somewhat from those disclosed by Benner. Preferably, the total charge ratio is between about 0.7 and about 1.2 moles sulfuric acid per mole of nitrobenzene (fresh plus recycled), with approximately 0.9 moles sulfuric acid per mole nitrobenzene being approximately optimum. P-aminophenol liquor (aqueous phase) leaving the last hydrogenator should contain at least about 5% free acid.

Preferably, the platinum catalyst is provided on a carbon support at a level of approximately 0.5-5% by weight. Other acid resistant supports may also be used, but abrasive supports should be avoided when ceramic glass-lined reactors are used. Platinum oxide catalysts are also suitable but platinum metal is preferred.

Catalyst loading is also a parameter of some importance in the process of the invention. Preferably, the charge mixture contains between about $3.0 \times 10^{-5}$ and about $5.0 \times 10^{-4}$ gram-atoms platinum per mole of nitrobenzene substrate.

As indicated above, the sulfur compound is supplied to the process at a rate sufficient to provide a concentration of about 0.1 to about 100 moles, preferably about 0.1 to 10 moles per gram atom of platinum metal. Optimum sulfur compound content varies inversely with the molecular weight of the sulfur compound, but essentially always falls within the aforesaid ranges. In a continuous operation such as that described above, the sulfur compound may be added in small increments at regular intervals to provide the approximate amounts needed, and the reaction mixture monitored so that the addition schedule can be modified if the sulfur compound content is moving out of the desired range. Because formation of by-product aniline is essentially eliminated, hydrogen flow to the continuous process adjusts to approximately two moles per mole of fresh nitrobenzene charged to the reaction.

A cationic surfactant is preferably included in the charge mixture to facilitate extraction of phenylhydroxylamine into the aqueous phase. Various quaternary ammonium salts or hydroxides can be used. Typically, the surfactant is present in a proportion of 0.1 to 1% by weight based on the total reaction mass.

As noted above, the process of the invention is useful for the preparation of both unsubstituted and lower alkyl substituted p-aminophenols, by catalytic hydrogenation of the corresponding unsubstituted or substituted nitrobenzene. Thus, generally the substrate used in the reaction may have the formula

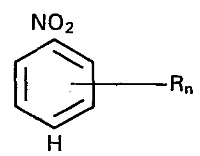

where R is lower alkyl and n is 0, 1 or 2. Typical of substrates which are useful in the process of the invention are o-nitrotoluene, m-nitrotoluene and 2,6-dimethylnitrobenzene.

Although the process of the invention is advantageously and preferably carried out in a continuous

system such as the cascade reactor system illustrated in the drawing, it will be understood that batch systems may also be used. In such event, the nitroaryl substrate, catalyst, acid and sulfur compound are charged to the batch hydrogenator in such amounts as to provide the proportions referred to above. After reaction is carried to the desired conversion, the batch is blown or pumped to a decant tank where the nitroaryl phase containing the catalyst separates from the aqueous phase containing the p-aminophenol. Nitroaryl substrate and catalyst are recycled to the reactor and the aqueous phase is subjected to further conventional treatment for purification and recovery of the p-aminophenol product.

The following examples illustrate the invention.

*Example 1*

Nitrobenzene was converted to p-aminophenol by catalytic hydrogenation in the presence of dilute sulfuric acid, using a 1 liter Morton flask fitted with an agitator, thermometer, reflux condenser and low pressure nitrogen and hydrogen feed lines.

A charge mixture was prepared by adding the following components, in the order given, to the Morton flask:

Nitrobenzene (57 ml)
Deionized water (406 ml)
Concentrated sulfuric acid (27 ml)
3% platinum on carbon catalyst, 70% wet (2.0 grams)
Arquad 12-33 dodecyltrimethylammonium chloride cationic surfactant (30% solution in isopropanol) (1.71 ml)
Diethyl sulfide (0.00846 grams).

After the charge mixture was prepared in the flask, the flask was purged with nitrogen and then with hydrogen. The charge mixture was then heated under mild agitation to a temperature of 80°C after which the agitator speed was increased and hydrogen absorption began. Hydrogen pressure was maintained at approximately 5 inches water and the temperature was controlled at 78-82°C throughout the reaction period. After three hours, approximately 75% of the nitrobenzene had reacted, at which point the reaction was terminated and the reactor flask purged with nitrogen.

To facilitate yield determination, the catalyst was recovered by filtration, and the filtered reaction mixture thereafter placed in a separatory funnel where the organic and aqueous phases were allowed to separate. The unreacted nitrobenzene organic phase was removed and weighed. The aqueous phase was neutralized with ammonia to a pH of 7.5-8.0, thereby precipitating the p-aminophenol product. This product was recovered by filtration, washed and dried for yield determination. Nitrobenzene conversion was calculated by subtracting the weight of unreacted nitrobenzene from the nitrobenzene charge and dividing by the latter. Selectivity of the diethyl sulfide modified catalyst system was determined by dividing the moles of p-aminophenol formed by the moles of nitrobenzene consumed, and multiplying by 100. Taking into consideration known solubility losses, selectivity of the reaction was determined to be about 78.9% at a conversion of 75%.

To provide a basis of comparison, another reaction was carried out in substantially the same way as described above except that no sulfur compound additive was included in the charge mixture. At a nitrobenzene conversion of 69.2%, selectivity for p-aminophenols was determined to be only 68.2%.

*Example 2*

Using the apparatus and method generally described in Example 1, a series of catalytic hydrogenations was carried out at 80°C for conversion of nitrobenzene to p-aminophenol. In the first run of this series no catalyst modifier was used while, in the other runs, various divalent sulfur compounds were employed in varying amounts as catalyst modifiers. The reaction mixture was worked up in essentially the same way as in Example 1 and the conversion, selectivity and p-aminophenol productivity were determined. The results of the runs of this Example are set forth in Table 1.

*Table 1*

*At 80°C*

| Catalyst Modifier | Modifier Moles g-atom Pt. | Conversion | Selectivity | g's PAP hr. | Time |
|---|---|---|---|---|---|
| None | — | 69.2% | 68.2% | 9.54 | 180 min. |
| $(CH_3)_2S$ | 16.9 | 33.3 | 71.7 | 4.58 | 190 min. |
| $(C_2H_5)_2S$ | 1.0 | 74.6 | 78.9 | 11.92 | 180 min. |
| dl-methionine | 0.5 | 80.8 | 72.1 | 11.76 | 180 min. |
| $(C_2H_5)_2S$ | 9.2 | No reaction | | | |

*Example 3*

Using the apparatus and method generally described in Example 1, another series of catalytic hydrogenations of nitrobenzene to p-aminophenol was carried out at 95°C. In one of these runs, no sulfide compound was used while, in the others, a divalent sulfur compound was incorporated in the charge mixture to enhance catalyst selectivity. Results of the runs of this Example are set forth in Table 2.

*Table 2*

*At 95°C*

| Catalyst Modifier | Modifier Moles g-atom Pt. | Conversion | Selectivity | g's PAP hr. | Time |
|---|---|---|---|---|---|
| $(CH_3)_2S$ | 1.2 | 28.4% | 79.2% | 4.55 | 180 min. |
| $(C_2H_5)_2S$ | 1.0 | 27.2 | 87.7 | 4.81 | 180 min. |
| None | — | 35.6 | 74.0 | 4.92 | 195 min. |

In view of the above, it will be seen that the several objects of the invention are achieved and other advantageous results attained.

As various changes could be made in the above methods without departing from the scope of the invention, it is intended that all matter contained in the above description or shown in the accompanying drawings shall be interpreted as illustrative and not in a limiting sense.

## Claims

1. A process for the preparation of substituted or unsubstituted p-aminophenol having the formula

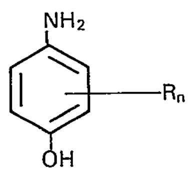

where R is lower alkyl and n is 0, 1 or 2, the process comprising the steps of:
preparing a charge mixture comprising a substrate comprising a substituted or unsubstituted nitrobenzene having the formula

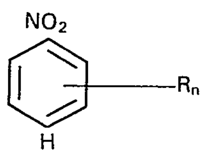

where R and n are as defined above, a highly dissociated acid, a catalyst containing platinum, and a divalent sulfur compound in which sulfur is bonded in two other moieties, said sulfur compound being present in a proportion of from 0.1 to 100 moles per gram atom of platinum, said mixture comprising an organic phase containing said substrate and an aqueous phase containing said acid;
introducing hydrogen into the mixture at a pressure of from to 4.447 bar (0 to 50 psig) while agitating said mixture at a temperature of from 70°C to 100°C, thereby causing said substrate to be reduced to a hydroxylamine having the formula

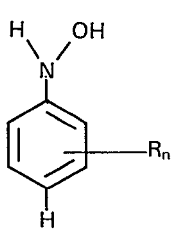

where R and n are defined above, said hydroxylamine being rearranged in the presence of said acid to said substituted or unsubstituted p-aminophenol; and recovering the p-aminophenol product from the aqueous phase.

2. A process as set forth in claim 1 wherein the hydrogenation reaction is terminated after a reduction of between 50 and 90% of the substrate contained in the charge mixture, and the aqueous phase containing the substituted or unsubstituted p-aminophenol product is separated from the organic phase containing the catalyst and unreacted substrate.

3. A process as set forth in claim 2 wherein the organic phase containing catalyst and unreacted substrate is recycled and mixed with additional amounts of substrate, dilute acid solution and sulfur compound to provide a charge mixture subject to further hydrogenation.

4. A process as set forth in claim 1, 2, or 3 wherein said charge mixture contains between 0.1 and 10 moles sulfur compound per gram atom of platinum.

5. A process as set forth in any one of claims 1 to 4 wherein said acid is selected from sulfuric acid, phosphoric acid, trihaloacetic acid, benzenesulfonic acid and toluenesulfonic acid.

6. A process as set forth in claim 5 wherein said acid comprises sulfuric acid.

7. A process as set forth in claim 6 wherein said charge mixture initially contains between 0.7 and 1.2 moles sulfuric acid per mole of substrate.

8. A process as set forth in any one of the preceding claims wherein said acid, said substrate and said catalyst are continuously introduced into a hydrogenation vessel and the products of the hydrogenation reaction are continuously removed therefrom.

9. A process as set forth in any one of the preceding claims wherein the hydrogenation reaction is carried out in a plurality of hydrogenation vessels arranged in series.

10. A process as set forth in claim 9 wherein the reaction mixture discharged from the last of said series of hydrogenation vessels is transferred to a separation vessel where said aqueous phase and said organic phase are continuously separated and said organic phase continuously returned to the first of said series of hydrogenation vessels.

11. A process as set forth in any one of the preceding claims wherein said substrate is selected from the group consisting of nitrobenzene, o-nitrotoluene, m-nitrotoluene and 2,6 dimethylnitrobenzene.

12. A process as set forth in any one of the preceding claims wherein said sulphur compound is selected from the group consisting of dialkyl sulfides, diaryl sulfides, dialkyl disulfides, aryl alkyl sulfides, thiophene, substituted thiophenes, thiophane, substituted thiophanes, aliphatic thiols, aromatic thiols, methionine and hydrogen sulfide.

13. A process as set forth in claim 12 wherein said sulfur compound comprises methionine.

14. A process as set forth in claim 12 wherein said sulfur compound comprises diethyl sulfide.

15. A process according to any one of the preceding claims wherein said sulfur compound has an atmospheric boiling point not substantially lower than the temperature at which the catalytic hydrogenation is carried out.

**Patentansprüche**

1. Verfahren zur Herstellung eines substituierten oder unsubstituierten p-Aminophenols der Formel

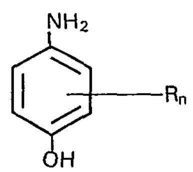

worin R niederes Alkyl und n 0, 1 oder 2 ist, bei dem man ein Beschickungsgemisch aus einem Substrat, das ein substituiertes oder unsubstituiertes Nitrobenzol der Formel

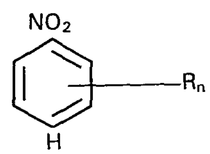

aufweist, worin R und n wie oben angegeben definiert sind, einer stark dissoziierten Säure, einem Platin enthaltenden Katalysator und einer zweiwertigen Schwefelverbindung herstellt, in welcher der Schwefel an zwei andere Verbindungsteile gebunden ist und die in einem Verhältnis von 0,1 bis 100 Mole je Grammatom Platin vorliegt, wobei dieses Gemisch eine das Substrat enthaltende organische Phase und eine die Säure enthaltende wässrige Phase aufweist,
in das Gemisch bei einem Druck von 1 bis 4,447 bar (0 bis 50 psig) unter Rührung bei einer Temperatur von 70°C bis 100°C Wasserstoff einführt und dadurch das Substrat zu einem Hydroxylamin der Formel

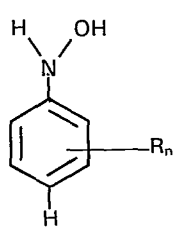

reduziert, in der R und n wie oben definiert sind, wobei das Hydroxylamin in Gegenwart der Säure zu dem substituierten oder unsubstituierten p-Aminophenol umgelagert wird, und
das p-Aminophenol-Produkt aus der wässrigen Phase gewinnt.

2. Verfahren nach Anspruch 1, bei dem man die Hydrierreaktion beendet, nachdem zwischen 50 und 90% des in dem Beschickungsgemisch enthaltenen Substrats reduziert sind, und die das substituierte oder unsubstituierte p-Aminophenol-Produkt enthaltende wässrige Phase von der den Katalysator und das nicht umgesetzte Substrat enthaltenden organischen Phase abtrennt.

3. Verfahren nach Anspruch 2, bei dem man die den Katalysator und nicht umgesetztes Substrat enthaltende organische Phase zurückführt und mit zusätzlichen Mengen Substrat, verdünnter Säure-Lösung und Schwefelverbindung unter Bildung eines Beschickungsgemisches mischt, das weiterer Hydrierung unterworfen wird.

4. Verfahren nach Anspruch 1, 2 oder 3, bei dem das genannte Beschickungsgemisch zwischen 0,1 und 10 Mole Schwefelverbindung je Grammatom Platin enthält.

5. Verfahren nach einem der Ansprüche 1 bis 4, bei dem man die genannte Säure unter Schwefelsäure, Phosphorsäure, Trihalogenessigsäure, Benzolsulfonsäure und Toluolsulfonsäure auswählt.

6. Verfahren nach Anspruch 5, bei dem die genannte Säure Schwefelsäure aufweist.

7. Verfahren nach Anspruch 6, bei dem das Beschickungsgemisch zu Anfang zwischen 0,7 und 1,2 Mole Schwefelsäure je Mol Substrat enthält.

8. Verfahren nach einem der vorhergehenden Ansprüche, bei dem man die Säure, das Substrat und den Katalysator in einen Hydrierbehälter kontinuierlich einführt und die Produkte der Hydrierungsreaktion kontinuierlich daraus entfernt.

9. Verfahren nach einem der vorhergehenden Ansprüche, bei dem man die Hydrierreaktion in mehreren, hintereinander angeordneten Hydrierungsbehältern durchführt.

10. Verfahren nach Anspruch 9, bei dem man das aus dem letzten Behälter der Reihe von Hydrierungsbehältern abgegebene Reaktionsgemisch in einen Trennbehälter überführt, in dem man die wässrige Phase und die organische Phase kontinuierlich trennt und die organische Phase kontinuierlich zu dem ersten Behälter der Reihe von Hydrierungsbehältern zurückführt.

11. Verfahren nach einem der vorhergehenden Ansprüche, bei dem man das Substrat aus der aus Nitrobenzol, o-Nitrotoluol, m-Nitrotoluol und 2,6-Dimethylnitrobenzol bestehenden Gruppe auswählt.

12. Verfahren nach einem der vorhergehenden Ansprüche, bei dem man die Schwefelverbindung aus der aus Dialkylsulfiden, Diarylsulfiden, Dialkyldisulfiden, Arylalkylsulfiden, Thiophen, substituierten Thiophenen, Thiophan, substituierten Thiophanen, aliphatischen Thiolen, aromatischen Thiolen, Methionin und Schwefelwasserstoff bestehenden Gruppe auswählt.

13. Verfahren nach Anspruch 12, bei dem die Schwefelverbindung Methionin aufweist.

14. Verfahren nach Anspruch 12, bei dem die Schwefelverbindung Diäthylsulfid aufweist.

15. Verfahren nach einem der vorhergehenden Ansprüche, bei dem die Schwefelverbindung einen atmosphärischen Siedepunkt hat, der nicht wesentlich tiefer als die Temperatur liegt, bei der die katalytische Hydrierung durchgeführt wird.

**Revendications**

1. Procédé pour la préparation d'un p-aminophénol substitué ou non substitué répondant à la formule

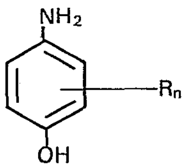

dans laquelle R est un alkyle inférieure et n est 0, 1 ou 2, le procédé comprenant les stades de: préparation d'une charge mélangée comprenant un substrat comprenant un nitrobenzène substitué ou non substitué, répondant à la formule

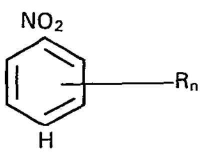

dans laquelle R et n sont comme défini ci-dessus, un acide fortement dissocié, un catalyseur contenant du platine et un composé du soufre divalent, dans lequel le soufre est uni à deux autres fragments, ledit composé du soufre étant présent en une proportion de 0,1 à 100 moles par atome-gramme de platine, ledit mélange comprenant une phase organique contenant ledit substrat et une phase aqueuse contenant ledit acide; introduction d'hydrogène dans le mélange à une pression de 1 à 4,447 bars (0 à 50 psig) avec agitation dudit mélange à une température de 70°C à 100°C pour provoquer la réduction dudit substrat en une hydroxylamine répondant à la formule

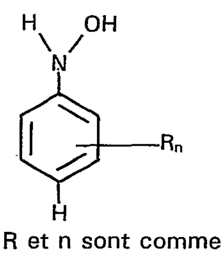

dans laquelle R et n sont comme défini ci-dessus, ladite hydroxylamine étant transposée en présence dudit acide en ledit p-aminophénol substitué ou non substitué; et récupération du p-aminophénol produit à partir de la phase aqueuse.

2. Procédé comme indiqué dans la revendication 1, dans lequel on arrête la réaction d'hydrogénation après une réduction entre 50 et 90% du substrat contenu dans le mélange réactionnel et on sépare la phase aqueuse, contenant le p-aminophénol substitué ou non substitué produit, d'avec la phase organique contenant le catalyseur et le substrat n'ayant pas réagi.

3. Procédé comme indiqué dans la revendication 2 dans lequel la phase organique, contenant le catalyseur et le substrat n'ayant pas réagi, est recyclée et mélangée avec des quantités additionnelles de substrat, de solution diluée d'acide et de composé sulfuré pour fournir une charge mélangée soumise à une nouvelle hydrogénation.

4. Procédé comme indiqué dans les revendications 1, 2 ou 3 dans lequel ladite charge mélangée contient entre 0,1 et environ 10 moles de composé sulfuré par atome-gramme de platine.

5. Procédé comme indiqué dans l'une quelconque des revendications 1 à 4 dans lequel ledit acide est choisi parmi l'acide sulfurique, l'acide phosphorique, un acide trihalogéno-acétique, l'acide benzènesulfonique et l'acide toluènesulfonique.

6. Procédé comme indiqué dans la revendication 5 dans lequel ledit acide comprend de l'acide sulfurique.

7. Procédé comme indiqué dans la revendication 6 dans lequel ladite charge mélangée contient initialement entre 0,7 et 1,2 mole d'acide sulfurique par mole de substrat.

8. Procédé comme indiqué dans l'une quelconque des revendications précédentes dans lequel ledit acide, ledit substrat et ledit catalyseur sont introduits en continu dans un récipient d'hydrogénation et les produits de la réaction d'hydrogénation en sont éliminés en continu.

9. Procédé comme indiqué dans l'une quelconque des revendications précédentes dans lequel la réaction d'hydrogénation est effectuée dans plusieurs récipients d'hydrogénation disposés en série.

10. Procédé comme indiqué dans la revendication 9 dans lequel le mélange réactionnel évacué du dernier récipient d'hydrogénation de ladite série est transféré dans un récipient de séparation où ladite phase aqueuse et ladite phase organique sont séparées en continu et ladite phase organique est retournée en continu dans le premier des récipients d'hydrogénation de ladite série.

11. Procédé comme indiqué dans l'une quelconque des revendications précédentes dans lequel ledit substrat est choisi dans le groupe constitué par le nitrobenzène, l'o-nitrotoluène, le m-nitrotoluène et le 2,6-diméthylnitrobenzène.

12. Procédé comme indiqué dans l'une quelconque des revendications précédentes dans lequel ledit composé sulfuré est choisi dans le groupe constitué par les sulfures de dialkyle, les sulfures de diaryle, les disulfures de dialkyle, les sulfures d'arylalkyle, le thiophène, les thiophènes substitués, le thiophane, les thiophanes substitués, les thiols aliphatiques, les thiols aromatiques, la méthionine et le sulfure d'hydrogène.

13. Procédé comme indiqué dans la revendication 12 dans lequel ledit composé sulfuré comprend de la méthionine.

14. Procédé comme indiqué dans la revendication 12 dans lequel ledit composé sulfuré comprend du sulfure diéthyle.

15. Procédé selon l'une quelconque des revendications précédentes dans lequel ledit composé sulfuré a un point d'ébullition atmosphérique qui n'est pas nettement inférieur à la température à laquelle l'hydrogénation catalytique est effectuée.

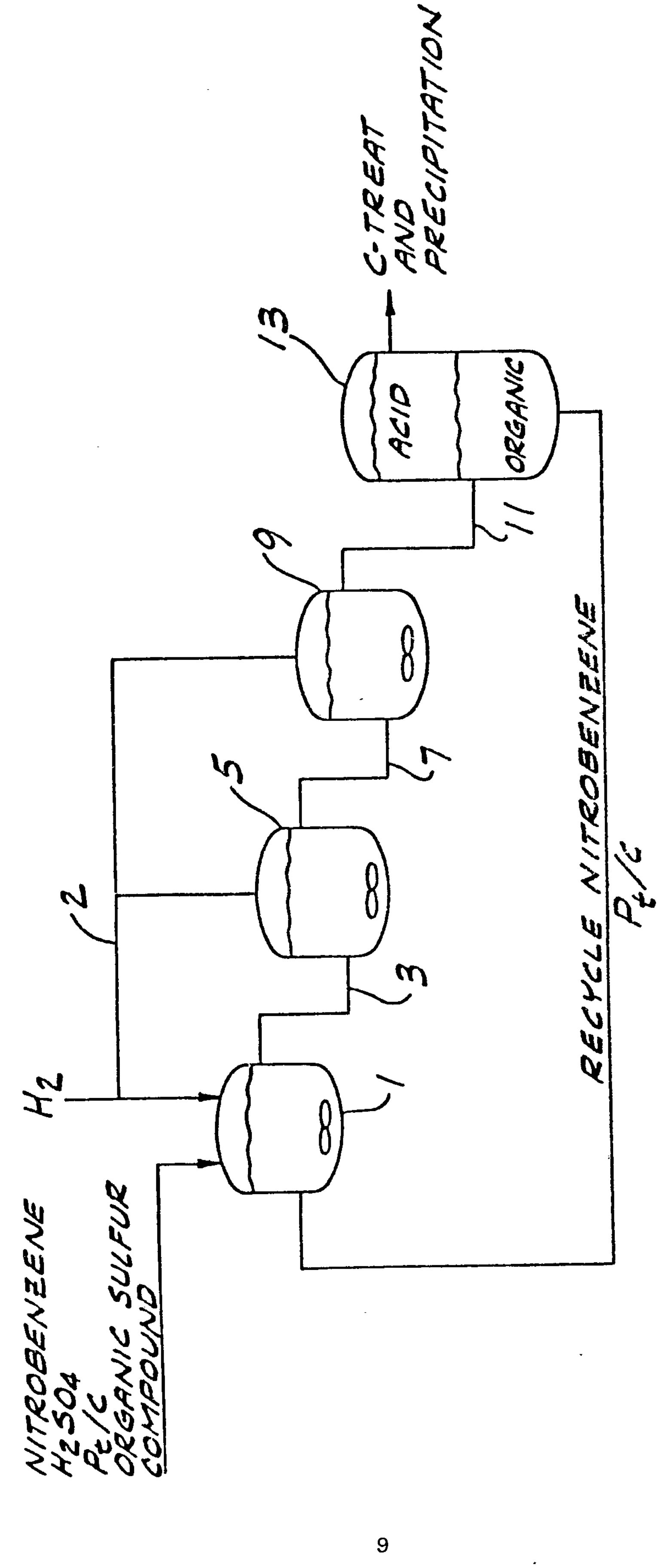
NITROBENZENE
H2SO4
Pt/C
ORGANIC SULFUR
COMPOUND
H2
1
2
3
5
7
9
11
13
ACID
ORGANIC
C-TREAT
AND
PRECIPITATION
RECYCLE NITROBENZENE
Pt/C